# EUROPEAN PATENT APPLICATION

(11) **EP 1 982 638 A1**
(43) Date of publication of application: **22.10.2008**
(21) Application number: 08007589.8
(22) Date of filing: 18.04.2008
(51) Int. Cl.: A61B 1/04

(54) **Endoscope image filing system**

(30) Priority: 20.04.2007 JP 2007111485
(71) Applicant: Olympus Corporation, Shibuya-ku Tokyo (JP)
(72) Inventor: Takizawa, Kazuhiro c/o Olympus Corporation, Tokyo (JP)
(74) Representative: Winter, Brandl, Fürniss, Hübner Röss, Kaiser, Polte Partnerschaft Patent- und Rechtsanwaltskanzlei

(57) **Abstract**

An endoscope image filing system includes: an original image file unit that stores the same digital image as that output from an electronic endoscope having an image capturing element and then input an image processing circuit; a library unit that stores a plurality of image processing software components; a selecting unit that selects one of the plurality of image processing software components stored in the library unit; and an image reproducing unit that processes the digital image stored in the original image file unit, according to the selected image processing software component, such that the digital image can be displayed.

## Description

### BACKGROUND OF THE INVENTION

### Field of the Invention

The present invention relates to an endoscope image filing system that stores digital images captured by an electronic endoscope including an image capturing element.

### Description of Related Art

In recent years, electronic endoscopes have come into widespread use in a medical field. Images captured by the electronic endoscope are stored in an external recording medium, such as photographic paper, a magnetic tape, and a magnetic disc, and are referred to when a doctor makes a diagnosis, if necessary. In order to observe a change in a diseased part of a patient with time, it is necessary to compare the previous image and the current image of the diseased part. However, when the images are captured by different types of endoscopes, it is difficult to perform exact comparison. A very small difference between the colors of the images captured by different types of endoscopes is likely to hinder the exact diagnosis of a doctor.

When observing the internal organs using the endoscope, the internal organs generally look reddish. The doctor can recognize a very small difference in the color of a diseased part from the observation images having basically the same color by experience.

Therefore, when a new type of endoscope is used, a small difference between the color of the image captured by the previous endoscope and the color of the image captured by the new type of endoscope is likely to have an effect on the diagnosis.

When a remote diagnosis using the endoscope is put to practical use in the near future, the doctor may need to make a diagnosis on the basis of the image captured by a different type of endoscope from the endoscope that is usually used. In this case, of course, the doctor should make an exact diagnosis.

Therefore, an apparatus capable of absorbing the difference between the images captured by different types of endoscopes is needed. As an example of the apparatus, Japanese Unexamined Patent Application Publication No. 8-238223 discloses a medical image display apparatus and a medical image filing system.

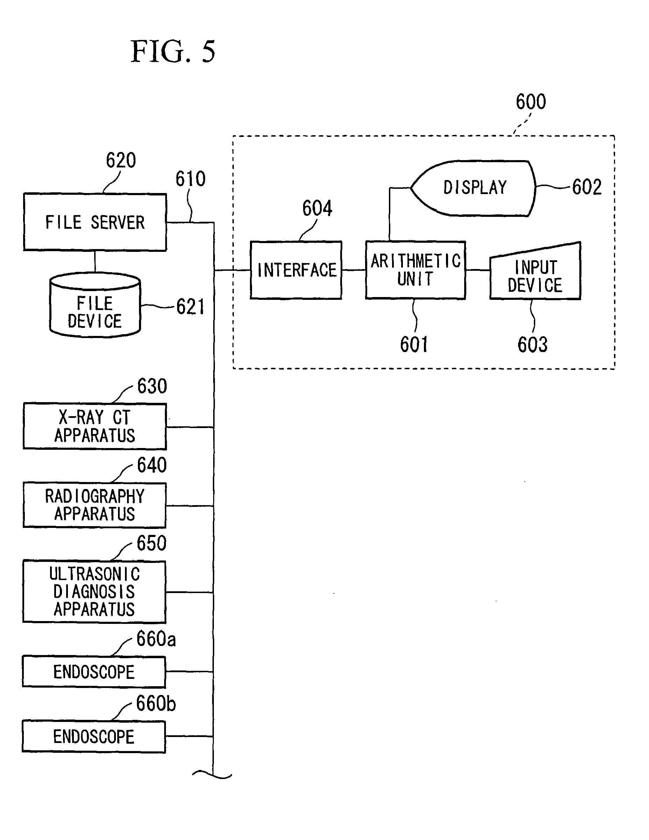

FIG. 5 shows the structure of the medical image display apparatus and the medical image filing system disclosed in Japanese Unexamined Patent Application Publication No. 8-238223. A medical image display apparatus 600 includes an arithmetic unit 601, a display 602, an input device 603, and an interface 604, and is connected to a network 610. In addition, endoscopes 660a and 660b, various examination apparatuses (an X-ray CT apparatus 630, a radiography apparatus 640, and an ultrasonic diagnosis apparatus 650), and a file server 620 for a medical image database are connected to the network 610. Medical images obtained by the examination apparatuses are stored in the file server 620 as medical image files.

When reading the medical image file and displaying the read file, the medical image display apparatus 600 performs color adjustment with reference to a preset display pallet table that is stored in a file device 621, and displays the adjusted color. The pallet table includes, for example, a red level, a green level, a blue level, and contrast. In addition, the pallet table is set for each examination apparatus by attribute data, such as an examination apparatus number, or the user may arbitrarily set the pallet table. In this way, it is possible for the user to set a user's favorite color for each type of examination apparatus. As a result, it is possible to display an image absorbing the difference between the colors of the images captured by different types of examination apparatuses.

The related art has performed a correction process on the final image output from the endoscope. However, during image processing, the original information of the original image is greatly damaged due to quantization errors in operation, which is not limited to the endoscope. Therefore, there are limitations in correcting image data subjected to various image processing. The image processing of the endoscope is specialized in the delicate color representation of red or yellow, which is a main color of the internal organs. A simple process of adjusting the red, green, and blue levels of the image is likely to damage the delicate color representation of the original image. In addition, since the adjustment is performed on the basis of an ambiguous standard, such as the user's preference, it is difficult to absolutely depend on the adjusted image during the diagnosis.

The invention has been made in an effort to solve the above problems, and an object of the invention is to provide an endoscope image filing system that, even when an image is captured by a different type of image capturing device from a specific type of image capturing device, is capable of acquiring an image having the same quality as that captured by the specific type of image capturing device.

### SUMMARY OF THE INVENTION

In order to achieve the object, according to an aspect of the invention, an endoscope image filing system includes: an original image file unit that stores the same digital image as that output from an electronic endoscope having an image capturing element and then input an image processing circuit; a library unit that stores a plurality of image processing software components; a selecting unit that selects one of the plurality of image processing software components stored in the library unit; and an image reproducing unit that processes the digital image stored in the original image file unit, according to the selected image processing software component, such that the digital image can be displayed.

Preferably, the endoscope image filing system according to the above-mentioned aspect further includes a display unit that displays the digital image processed by the image reproducing unit.

According to the above-mentioned aspect of the invention, before image processing is performed, a digital image that does not depend on the type of image capturing device is stored in the original image file unit, and the image reproducing unit performs image processing on the digital image according to the image processing software component selected by the selecting unit. Therefore, even when an image is captured by a different type of image capturing device, it is possible to acquire an image having the same quality as that captured by the same type of image capturing device.

### BRIEF DESCRIPTION OF THE DRAWINGS

FIG. 1 is a block diagram illustrating the structure of an endoscope image filing system according to a first embodiment of the invention.
FIG. 2 is a diagram illustrating the format of an original image file according to the first embodiment of the invention.
FIG. 3 is a block diagram illustrating the structure of an endoscope system according to a second embodiment of the invention.
FIG. 4 is a block diagram illustrating the structure of an endoscope image filing system according to a third embodiment of the invention.
FIG. 5 is a block diagram illustrating the structure of a medical image display apparatus and a medical image filing system according to the related art.

### DETAILED DESCRIPTION OF THE INVENTION

Hereinafter, exemplary embodiments of the invention will be described with reference to the accompanying drawings.

### First embodiment

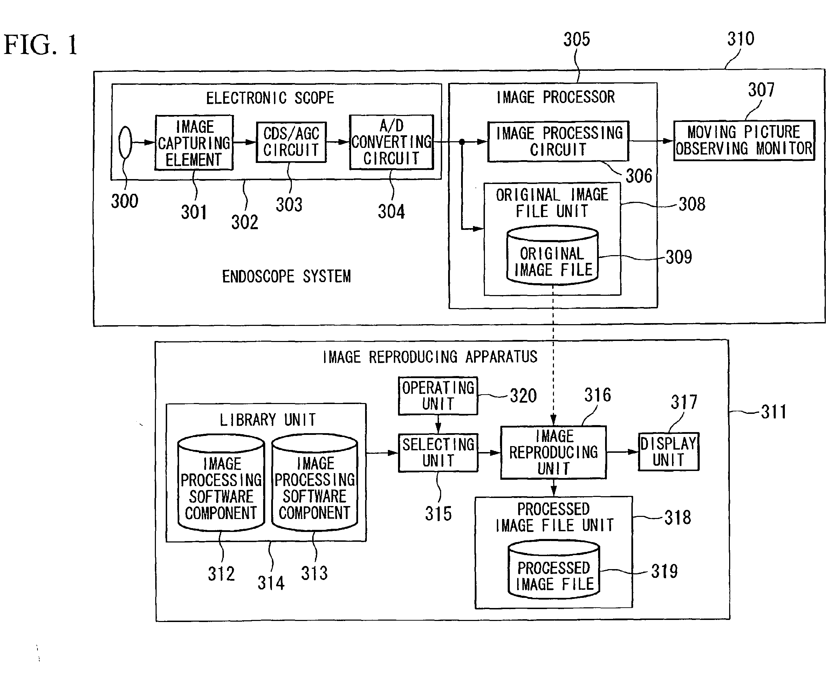

First, a first embodiment of the invention will be described. FIG. 1 is a block diagram illustrating the structure of an endoscope image filing system according to this embodiment. The endoscope image filing system according to this embodiment includes an endoscope system 310 and an image reproducing apparatus 311.

First, the structure of the endoscope system 310 will be described. The endoscope system 310 includes an electronic scope 302 (electronic endoscope), an image processor 305, and a moving picture observing monitor 307. The electronic scope 302 includes an objective optical system 300, a solid-state image capturing element 301, a CDS/AGC circuit 303, and an A/D converting circuit 304. The objective optical system 300 forms the image of an object. The solid-state image capturing element 301 includes, for example, a CCD, performs photoelectric conversion on the image of the object formed by the objective optical system 300, and outputs image signals. The CDS/AGS circuit 303 performs correlation double sampling (CDS) and automatic gain control (AGC) on the image signals output from the solid-state image capturing element 301. The A/D converting circuit 304 converts analog image signals output from the CDS/AGS circuit 303 into digital image signals.

The image processor 305 includes an image processing circuit 306 and an original image file unit 308. The image processing circuit 306 performs predetermined image processing on the digital image signals that are continuously input from the electronic scope 302 in real time to convert the digital image signals into images to be viewed. The original image file unit 308 stores the digital image signals received from the electronic scope 302 as an original image file 309 having a predetermined format. Moving pictures output from the image processor 305 are input to the moving picture observing monitor 307, such that the user operates the electronic scope 302 while viewing the image displayed on the moving picture observing monitor 307.

Next, the structure of the image reproducing apparatus 311 will be described. A library unit 314 stores a plurality of image processing software components 312 and 313 (image processing programs). These image processing software components are software components (programs) that perform image processing corresponding to the image processing circuit 306 of the image processor 305, and the image processing software components perform different processes according to the type of endoscope.

A selecting unit 315 selects one of the image processing software components according to instructions from the user, reads out the selected image processing software component from the library unit 314, and loads it to an image reproducing unit 316. The image reproducing unit 316 performs predetermined image processing on the original image file 309 to be displayed as an image, according to the image processing software component selected by the selecting unit 315. The process result of the image reproducing unit 316 is displayed as an image by a display unit 317.

A processed image file unit 318 stores the image processing result of the original image file 309 by the image reproducing unit 316 as a processed image file 319. An operating unit 320 includes a keyboard, a mouse, or switches for the user to select a desired image processing software component. The selecting unit 315 selects an image processing software component on the basis of a signal indicating the selection result of the image processing software component by the user that is output from the operating unit 320.

For the transmission of the original image file 309 between the endoscope system 310 and the image reproducing apparatus 311, the image reproducing apparatus 311 may communicate with the endoscope system 310 to receive the original image file 309 from the endoscope system 310, or the image reproducing apparatus 311 may read out the original image file 309 that is stored in an external recording medium, such as a memory card, by the endoscope system 310.

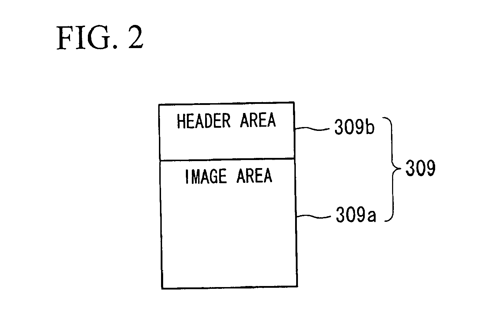

Next, the format of the original image file 309 will be described below. FIG. 2 shows an example of the format of the original image file 309. The original image file 309 includes an image area 309a that stores image data and a header area 309b that stores information appended to an image.

The electronic scope acquires images by the following methods: a method of acquiring images using RGB rotary filters (hereinafter, referred to as a rotary type); a method of acquiring images using three image capturing elements (hereinafter, referred to a three-element type); and a method of acquiring images using one image capturing element having a color filer (a primary color filter or a complementary color filter) adhered thereto (hereinafter, referred to as a single element type). In addition, the image capturing elements have different number of pixels or different lengths of effective bits. Therefore, it is necessary to record the storage format of data in the file.

Further, in order to adjust the white balance, it is necessary to capture the image of a white object before image capturing. It is preferable to also store a white balance coefficient acquired at that time. In this embodiment, the type of electronic scope (a rotary type, a three-element type, or a single element type), the total number of pixels, the length of valid bits, and the white balance coefficient are stored in the header area 309b. The model number of the image capturing element provided in the electronic scope may be recorded.

Next, the operation of the endoscope image filing system according to this embodiment will be described. An observation image is input to the image processor 305 as digital image signals through the objective optical system 300, the solid-state image capturing element 301, the CDS/AGC circuit 303, and the A/D converting circuit 304.

The input digital image signals are converted into an image to be viewed by the image processing circuit 306, and then output to the moving picture observing monitor 307. Then, the observation image is displayed on the moving picture observing monitor 307.

The user views the image displayed on the moving picture observing monitor 307, operates the electronic scope 302 to find a lesion location, and instructs the image processor 305 to capture an image of the lesion. In this case, digital image signals from the electronic scope 302 are stored as the original image file 309 by the original image file unit 308.

When reproducing the stored images, the user operates the operating unit 320 to instruct the selecting unit 315 to select an image processing software component to be used (an image processor to perform image processing). The selecting unit 315 reads the image processing software component selected by the user from the library unit 314, and loads the read image processing software component to the image reproducing unit 316. The image reproducing unit 316 performs predetermined image processing on data of the original image file 309 according to the image processing software component selected by the selecting unit 315, and outputs the process result to the display unit 317. In addition, the image processed by the image reproducing unit 316 is stored as the processed image file 319 by the processed image file unit 318, if necessary.

As described above, before image processing is performed, a digital image that does not depend on the type of endoscope is stored as the original image file 309 in the original image file unit 308, and the image reproducing unit 316 performs image processing on the original image file 309 according to the image processing software component selected by the selecting unit 315. Therefore, even when an image is captured by a different type of image capturing device from a specific type of image capturing device, it is possible to acquire an image having the same quality as that captured by the specific type of image capturing device. In this way, it is possible to consistently make a clinical chart regardless of the type of image capturing device and thus improve reliability in a remote diagnosis. Further, since the display unit 317 displays the image processed by the image reproducing unit 316, the user can visually check the image processing result by the image reproducing unit 316.

For example, when the clinical chart of a patient is stored as an image processed by an image processor A and an image processed by another image processor B is mixed with the image processed by the image processor A, the consistency of the clinical charts is not obtained. However, according to this embodiment, even when the image processor A cannot be used due to a change in the type of image capturing device, the original image captured by the image processor B is processed by an image processing software component that executes the same process as that of the image processor A, thereby obtaining the same image as that processed by the image processor A. Therefore, the user can make a consistent diagnosis with reference to the past image of the clinical chart, even when the type of endoscope is changed.

Furthermore, according to this embodiment, the original image before image processing is stored, and thus the flexibility of image processing performed on the image data is improved. Therefore, it is possible to generate an image that seems to be captured by a different type of image capturing device from the image capturing device that actually captures the image. Further, the image processing software component used to reproduce the image strictly reproduces the process performed by the existing image processor, which makes it possible to remove ambiguity, such as the subjectivity of the user. As a result, it is possible to improve the reliability of diagnosis.

The endoscope system 310 shown in FIG. 1 stores only the original image, but the invention is not limited thereto. As in a general endoscope, the output image from the image processing circuit 306 may be stored. For example, only the original image, both the original image and the processed image, or only the processed image may be stored such that the user can select a proper storage method. In addition, the library unit 314 shown in FIG. 1 stores two kinds of image processing software components, but it may store three or more image processing software components.

### Second embodiment

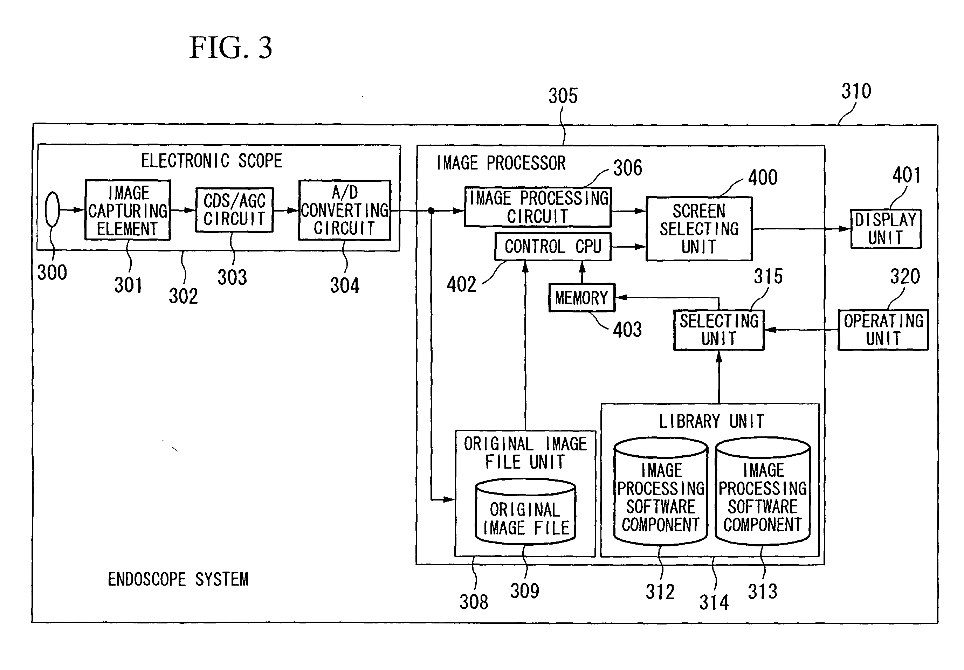

Next, a second embodiment of the invention will be described. FIG. 3 is a block diagram illustrating the structure of an endoscope system (endoscope image filing system) according to this embodiment. In FIG. 3, a control CPU 402 controls the overall operation of the endoscope system 310 in a moving picture observation mode, while it serves as an image reproducing unit in an image reproduction mode. A screen selecting unit 400 outputs a moving picture from the image processing circuit 306 (moving picture observation mode), or outputs the image processed by the control CPU 402 (image reproduction mode) according to an operation mode that is set by instructions from the user. A display unit 401 displays the image output from the screen selecting unit 400.

Next, the operation of the endoscope system 310 according to this embodiment will be described. The capturing of the original image file is the same as that in the first embodiment, and thus a description thereof will be omitted. During an image reproducing process, similar to the first embodiment, the selecting unit 315 selects an image processing software component from the library unit 314, on the basis of a signal indicating the selection result of the image processing software component by the user, which is output from the operating unit 320, and loads the selected image processing software component to a memory 403 for storing programs of the control CPU 402. The control CPU 402 reads the original image file 309 from the original image file unit 308, and performs image processing according to the image processing software component stored in the memory 403.

The image processed by the control CPU 402 is input to the screen selecting unit 400. The screen selecting unit 400 selects a reproduction image from the control CPU 402, and outputs the selected reproduction image to the display unit 401 in the image reproduction mode. The control CPU 402 cannot output image data at the timing when the display unit 401 immediately displays the image data, from the viewpoint of software process characteristics. Therefore, the screen selecting unit 400 includes a buffer memory that temporarily stores image data and a structure that outputs an image to the display unit 401 at the timing when the display unit 401 can display the image.

The image processing circuit 306 processes moving pictures in real time, and it is difficult for the CPU to perform the same process using a software component. However, since still pictures are processed in the image reproduction mode, the operation of the system does not fail even when the CPU performs an operation.

In the endoscope system 310 according to this embodiment, in the moving picture observation mode, the user needs to view the image on the display unit 401 and to determine whether to photograph a diseased part (store the image of the diseased part).

However, in the moving picture observation mode, the image displayed on the display unit 401 has been subjected to image processing by the image processing circuit 306 provided in the image processor 305. Therefore, when an inexperienced user discriminates the color of the processed image, the user cannot exactly determine the color of the processed image. In this case, the captured image is stored as the original image file 309, and then predetermined image processing is performed to convert the color of the image, thereby making a diagnosis on the basis of the converted color.

In the first embodiment, as shown in FIG. 1, the endoscope system 310 is separately provided from the image reproducing apparatus 311, and needs to transmit the original image file 309 to the image reproducing apparatus 311 using an external recording medium or a communication function, in order to convert the image. Therefore, it is difficult to perform image conversion while the electronic scope 302 is operated. In contrast, in this embodiment, since the image reproducing apparatus is integrated with the endoscope system 310, a file transmission process is not needed. Therefore, even when delicate decision is needed to determine whether to capture an image, it is possible to obtain an observation image having the same quality as that obtained by image processing performed by an experienced image processor. As a result, it is possible to reduce the possibility of missing a diseased part.

Furthermore, in this embodiment, the endoscope system 310 stores only the original image file 309, but the invention is not limited thereto. For example, similar to the first embodiment, if necessary, the image processing result by the control CPU 402 may be stored in the file. In addition, similar to the first embodiment, as in a general endoscope, the output image from the image processing circuit 306 may be stored. Further, similar to the first embodiment, two or more image processing software components may be stored in the library unit 314.

### Third embodiment

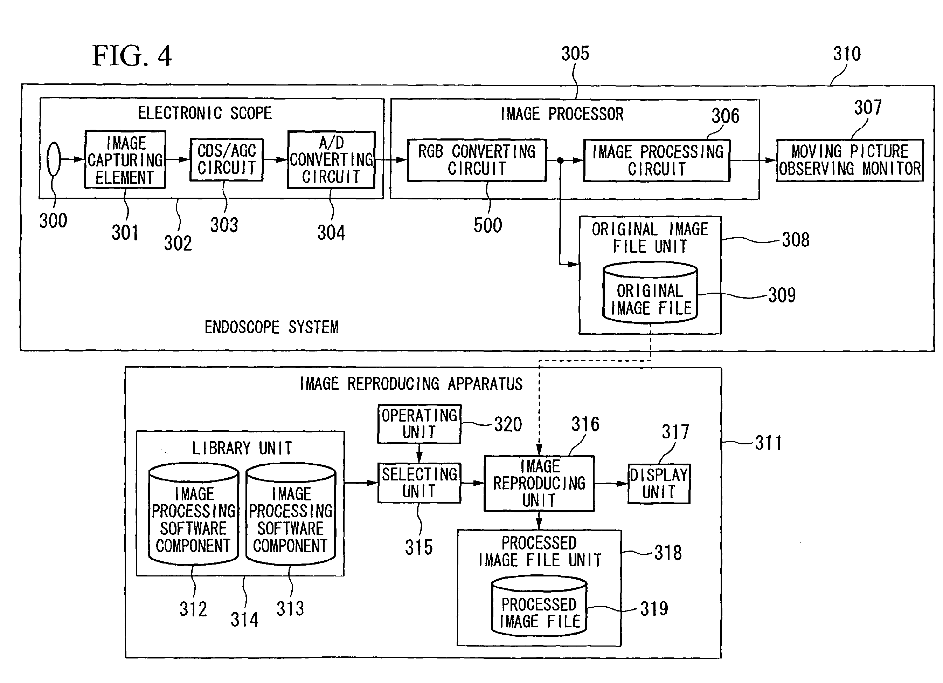

Next, a third embodiment of the invention will be described. FIG. 4 is a block diagram illustrating the structure of an endoscope image filing system according to this embodiment. In this embodiment, an RGB converting circuit 500 is provided in the previous stage of the image processing circuit 306 and the original image file unit 308. Image signals output from the electronic scope 302 are input to the image processor 305 in various formats, as described above. Even when a rotary electronic scope, a three-element electronic scope, and a single-element electronic endoscope have the same number of pixels, the amount of data of the image captured by the rotary or three-element electronic scope is different from the amount of data of the image captured by the single-element electronic scope. In addition, the color of the image captured by the single-element electronic scope depends on a color filter (a primary color and a complementary color). Therefore, in this embodiment, the RGB converting circuit 500 provided in the previous stage of the image processing circuit 306 and the original image file unit 308 converts the image captured by the single-element electronic scope into RGB signals of the same primary colors as those of the rotary electronic scope (and the three-element electronic scope). Of course, the RGB converting circuit 500 performs no process on the images captured by the rotary electronic scope and the three-element electronic scope. Alternatively, the RGB converting circuit 500 may convert complementary color image signals.

According to this embodiment, the original image file 309 stored in the original image file unit 308 has the same format regardless of the type of electronic scope. Therefore, the image reproducing unit 316 can simply acquire the image of the original image file 309. In addition, in FIG. 4, the RGB converting circuit 500 is provided in the endoscope system 310 shown in FIG. 1, but the invention is not limited thereto. The RGB converting circuit 500 may be provided in the endoscope system 310 shown in FIG. 3.

While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.

## Claims

1. An endoscope image filing system comprising:
an original image file unit that stores the same digital image as that output from an electronic endoscope including an image capturing element and then input an image processing circuit;
a library unit that stores a plurality of image processing software components;
a selecting unit that selects one of the plurality of image processing software components stored in the library unit; and
an image reproducing unit that processes the digital image stored in the original image file unit, according to the selected image processing software component, such that the digital image can be displayed.

2. The endoscope image filing system according to claim 1, further comprising:
a display unit that displays the digital image processed by the image reproducing unit.
